# FASCICULE DE BREVET EUROPEEN

(11) **EP 2 705 368 B1**
(45) Date de publication et mention de la délivrance du brevet: **19.08.2015**
(21) Numéro de dépôt: 12722545.6
(22) Date de dépôt: 27.04.2012
(51) Int. Cl.: G01N 33/569

(54) **UTILISATION DE EF-1 ET DE SES HOMOLOGUES, POUR DETECTER UNE PROTEINE RETROVIRALE P24 OU UN HOMOLOGUE DE CELLE-CI**
VERWENDUNG VON EF-1 UND HOMOLOGEN DAVON FÜR DEN NACHWEIS EINES P24-RETROVIRENPROTEINS ODER HOMOLOGS DAVON
USE OF EF-1 AND THE HOMOLOGUES THEREOF, TO DETECT A P24 RETROVIRAL PROTEIN OR A HOMOLOGUE OF SAME

(30) Priorité: 02.05.2011 FR 1153741
(43) Date de publication de la demande: 12.03.2014
(73) Titulaire: Apex Biosolutions, 25000 Besancon (FR); L'Universite De Franche Comte, 25030 Besancon (FR)
(72) Inventeur: HERBEIN, Georges, F-25320 Montferrand Le Chateau (FR); MOROT-BIZOT, Stéphanie, F-70190 Sorans les Breurey (FR)
(74) Mandataire: Nony
(86) Numéro de dépôt international: PCT/IB2012/052124
(87) Numéro de publication internationale: WO 2012/150535

(56) Documents cités:
- US-A1- 2004 005 548
- JOHNSON CRAIG M ET AL: "The NS5A protein of bovine viral diarrhoea virus interacts with the alpha subunit of translation elongation factor-1", JOURNAL OF GENERAL VIROLOGY, vol. 82, no. 12, décembre 2001 (2001-12), pages 2935-2943, XP002653923, ISSN: 0022-1317
- SIKORA D ET AL: "The hepatitis delta virus RNA genome interacts with eEF1A1, p54<nrb>, hnRNP-L, GAPDH and ASF/SF2", VIROLOGY, ACADEMIC PRESS,ORLANDO, US, vol. 390, no. 1, 20 juillet 2009 (2009-07-20), pages 71-78, XP027425238, ISSN: 0042-6822, DOI: DOI:10.1016/J.VIROL.2009.04.022 [extrait le 2009-05-22]
- KAWAGUCHI YASUSHI ET AL: "Interaction of herpes simplex virus 1 alpha regulatory protein ICP0 with elongation factor 1-delta: ICP0 affects translational machinery", JOURNAL OF VIROLOGY, vol. 71, no. 2, 1997, pages 1019-1024, XP002653924, ISSN: 0022-538X

## Description

La présente invention se rapporte au domaine du diagnostic biologique.

Plus précisément l'invention concerne l'utilisation d'une sous-unité d'une protéine Facteur d'Elongation de la traduction 1, ou EF-1, ou d'un homologue de celle-ci, ou d'une portion de ceux-ci, comme agent de capture pour la détection d'une protéine rétrovirale. L'invention concerne également un procédé et un kit de détection d'une protéine rétrovirale.

Le Syndrome de l'ImmunoDéficience Acquise (SIDA) est une maladie infectieuse due au Virus de l'Immunodéficience Humaine (VIH), qui se caractérise notamment par la destruction des cellules immunitaires chez l'individu infecté, et qui a fréquemment pour issue la mort du patient, des suites de maladies opportunistes.

Selon les estimations de l'United Nations Programme on HIV/AIDS (UNAIDS), plus de 33 millions de personnes dans le monde seraient atteintes du SIDA. Transmissible par voies sexuelle et sanguine, et également de la mère à l'enfant, cette maladie infecte 2 à 3 millions de nouvelles personnes chaque année. Très souvent, la transmission se fait alors que la personne infectée n'a pas été diagnostiquée comme telle, et n'a en conséquence pas pris les mesures de précaution nécessaires pour prévenir la contamination d'autrui. Un diagnostic tardif de cette maladie chez une personne nouvellement infectée est donc l'une des raisons majeures conduisant à l'augmentation et au renouvellement de la population mondiale infectée par le VIH.

Il est ainsi primordial de pouvoir diagnostiquer la présence du VIH chez un individu le plus rapidement possible après son infection.

Actuellement, beaucoup d'infections virales, et notamment le VIH, sont diagnostiquées par la détection de protéines virales et/ou d'anticorps ciblant ces protéines virales, dans un échantillon biologique, comme le sang, de l'individu testé. A titre d'exemple de protéines virales mises en oeuvre à cette fin, on peut citer la protéine rétrovirale p24, ou son homologue p26, issue du clivage de la polyprotéine GAG, et constitutive de la capside de certains virus, tel que le VIH-1, VIH-2 et les virus HTLV-1 (Human T-Lymphotropic Virus) et HTLV-2.

Le document US20040005548 décrit un procédé de diagnostique d'une infection par le VIH au moyen d'anticorps dirigés contre l'antigène p24 du VIH-1 et/ou contre l'antigène p26 du VIH2.

Les tests diagnostics commercialement disponibles pour la détection du VIH reposent, pour la plupart, sur la détection d'anticorps dirigés contre l'une des protéines du virus, p24, gp41, gp120, gp160 ou gp105. Ces tests présentent, toutefois, l'inconvénient de ne pas pouvoir donner un résultat positif avec un bon intervalle de confiance avant une période d'au moins 3 semaines à compter de la date présumée de l'infection. Cette période de temps est nécessaire au développement d'une réponse immunitaire suffisante pour qu'elle puisse être détectable.

D'autres tests dits de diagnostic rapide (TDR) ou de 4^{ème} génération (TDR 4G) s'appuient sur la détection combinée d'anticorps anti-VIH-1 ou anti-VIH-2 et de la protéine rétrovirale p24. Ils permettent l'obtention d'un résultat en environ 15 minutes. Cependant, leur sensibilité est relativement faible, et ils ne peuvent généralement détecter la protéine rétrovirale p24 à des concentrations inférieures à environ 50 pg/ml. Un résultat positif avec un intervalle de confiance raisonnable requiert la présence de la protéine rétrovirale p24 à des concentrations supérieures à environ 100 pg/ml. Ces tests peuvent donc donner de faux négatifs pour des patients infectés peu de temps avant le diagnostic, par exemple moins de 15 jours ou moins d'un mois. Ils doivent donc être renouvelés plusieurs semaines après l'obtention d'un résultat négatif afin de confirmer ou d'infirmer celui-ci. Ceci peut se traduire par une augmentation substantielle des coûts liés aux politiques sanitaires et de prévention de la maladie.

D'autres tests de diagnostic s'appuient sur la détection de la présence d'ADN ou d'ARN viraux, par exemple par PCR ou RT-PCR. Bien que de meilleure sensibilité que les tests précédents, ils sont d'un coût plus élevé, et nécessitent des locaux et une installation spécifiques. Ils sont donc peu accessibles aux pays en voie de développement n'ayant pas les ressources et les infrastructures adéquates à leur mise en place. Qui plus est, ils impliquent des manipulations précises et longues, habituellement 48 heures, entre le prélèvement de l'échantillon à tester et l'obtention du résultat.

Au vu de ce qui précède, il apparaît qu'il demeure un besoin de disposer d'un test de détection d'une infection virale qui soit rapide, simple, avec un seuil de sensibilité permettant un diagnostic précoce, et de faible coût.

Il demeure également un besoin de disposer d'un test de détection d'une infection virale fiable ne requérant la détection que d'une protéine rétrovirale.

Il existe encore un besoin de disposer d'un nouvel outil de détection de protéines rétrovirales qui soit robuste et de bonne sensibilité.

Il existe également un besoin de disposer d'un nouvel outil de capture de protéines rétrovirales.

Il existe encore un besoin de disposer d'un test de détection de protéines rétrovirales simple, rapide, reproductible, sensible, robuste et peu coûteux.

Il existe également un besoin de disposer d'un test de diagnostic permettant la détection précoce d'une infection virale.

Enfin, il existe un besoin d'un test de diagnostic du VIH fiable, rapide, sensible, robuste, reproductible et peu coûteux.

La présente invention a pour objet de satisfaire à ces besoins.

Selon un premier objet, la présente invention concerne l'utilisation d'au moins une sous-unité d'une protéine Facteur d'Elongation de la traduction 1, EF-1, d'un homologue de EF-1, ledit homologue de EF-1 présentant au moins 80% d'homologie avec EF-1 et une activité biologique similaire ou d'un fragment C-terminal de ceux-ci comprenant une séquence d'acides aminés variant de 150 à 250 acides aminés contigus de l'extrémité C-terminale de la sous-unité ou de son homologue, comme agent de détection d'une protéine rétrovirale p24, ou d'un homologue de celle-ci, ledit homologue de p24 présentant au moins 80% d'homologie avec p24 et une activité biologique similaire.

Sauf indications contraires, par la suite, lorsque le terme « EF-1 » est utilisé seul, il vise à désigner, indifféremment, la protéine EF-1, un homologue de EF-1, une sous-unité de ceux-ci, ou un fragment C-terminal d'une sous-unité.

De manière inattendue, et comme détaillé dans les exemples, les inventeurs ont observé que la protéine EF-1, et notamment la sous-unité EF-1α, peut lier une protéine rétrovirale p24 du VIH, et former avec elle un complexe protéique. Cette propriété est avantageusement mise à profit pour la capture et la détection de protéines rétrovirales. En outre, les inventeurs ont observé que l'association de EF-1 avec une polyprotéine virale GAG améliore la capture de la protéine p24, et par conséquent la sensibilité de la détection de cette protéine.

Ainsi, l'invention tire profit d'une propriété nouvellement observée de EF-1, à savoir sa forte affinité vis-à-vis des protéines rétrovirales, et plus particulièrement vis-à-vis de GAG et de p24. Cette propriété permet la détection, avec une bonne confiance, de la protéine rétrovirale p24 à de très faibles concentrations, par exemple inférieure à 100 pg/ml, voire inférieure à 50 pg/ml, voire encore inférieure à 3 pg/ml.

La présente invention permet donc de réaliser une détection précoce, sensible, rapide, fiable et peu coûteuse d'une infection par un virus exprimant une protéine p24, ou un homologue de celle-ci. Avantageusement, le diagnostic d'une infection virale au moyen d'une méthode selon l'invention peut ne requérir que la détection de la protéine rétrovirale p24, ou d'un homologue de celle-ci.

Selon un autre objet, la présente invention concerne un procédé pour détecter, dans un échantillon biologique, la présence présumée d'une protéine rétrovirale p24, ou d'un homologue de celle-ci, ledit homologue de p24 présentant au moins 80% d'homologie avec p24 et une activité biologique similaire, ledit procédé comprenant au moins les étapes consistant à :
a- mettre en contact ledit échantillon avec une quantité efficace d'au moins une sous-unité d'une protéine facteur d'élongation de la traduction 1, EF-1, d'un homologue de EF-1, ledit homologue de EF-1 présentant au moins 80% d'homologie avec EF-1 et une activité biologique similaire ou d'un fragment C-terminal de ceux-ci comprenant une séquence d'acides aminés variant de 150 à 250 acides aminés contigus de l'extrémité C-terminale de la sous-unité ou de son homologue, dans des conditions appropriées à la formation d'au moins un complexe protéique entre la sous-unité de EF-1, l'homologue de EF-1, ou le fragment C-terminal de ceux-ci, et au moins une protéine rétrovirale p24, ou l'homologue de celle-ci,
b- mettre en contact le complexe protéique de l'étape a- avec une entité bi-fonctionnelle, ladite entité étant dotée d'un groupement apte à se lier spécifiquement au moins à une protéine rétrovirale p24, ou l'homologue de celle-ci, dans ledit complexe, et d'un groupement apte à être détectable, directement ou indirectement, par un mode de détection, la mise en contact étant effectuée dans des conditions appropriées à l'établissement d'une liaison entre ladite protéine rétrovirale et ladite entité bi-fonctionnelle,
c- détecter ladite entité bi-fonctionnelle, la détection de ladite entité bi-fonctionnelle liée étant indicatrice de la présence de la protéine rétrovirale p24, ou de l'homologue de celle-ci.

Selon un mode de réalisation, une utilisation ou un procédé de l'invention peuvent être mis en oeuvre *in vivo, in vitro* ou *ex vivo,* et de préférence *in vitro* ou *ex vivo.*

Selon encore un autre objet, la présente invention concerne un kit de détection d'au moins une protéine rétrovirale p24, ou un homologue de celle-ci, ledit homologue de p24 présentant au moins 80% d'homologie avec p24 et une activité biologique similaire, comprenant :
- au moins un premier réceptacle comprenant au moins une sous-unité d'une protéine facteur d'élongation de la traduction 1, EF-1, un homologue de EF-1, ledit homologue de EF-1 présentant au moins 80% d'homologie avec EF-1 et une activité biologique similaire ou un fragment C-terminal de ceux-ci comprenant une séquence d'acides aminés variant de 150 à 250 acides aminés contigus de l'extrémité C-terminale de la sous-unité ou de son homologue, et
- au moins un second réceptacle comprenant une entité bi-fonctionnelle dotée d'un groupement apte à se lier spécifiquement au moins à ladite protéine rétrovirale p24, ou ledit homologue de celle-ci, susceptible d'être présent(e) dans un complexe protéique formé avec la sous-unité de EF-1, l'homologue de EF-1, ou le fragment C-terminal de ceux-ci, et un groupement apte à être détectable, directement ou indirectement, par un mode de détection.

Avantageusement, l'invention permet une amélioration de la sensibilité, de la rapidité et de la fiabilité du diagnostic d'infections par des virus exprimant une protéine rétrovirale p24, ou un homologue de celle-ci.

Avantageusement encore, l'invention permet de proposer un diagnostic précoce d'une infection par le VIH.

Avantageusement encore, l'invention permet de proposer des tests et des dispositifs pour le diagnostic d'infections par rétrovirus, peu coûteux, simples, rapides, et permettant de satisfaire aux différentes exigences dans le domaine, tant sur le plan économique que sur le plan de la santé publique et de la sécurité sanitaire. De tels tests peuvent permettre d'apporter une meilleure garantie concernant les transfusions sanguines humaines, d'établir un diagnostic précoce des infections virales, et d'optimiser les traitements thérapeutiques correspondants.

Au sens de l'invention, par « agent de détection » d'une molécule on entend désigner un composé doté de la propriété de lier, par exemple par affinité, ladite molécule lorsqu'il est mis en contact avec celle-ci dans des conditions appropriées.

Selon l'invention, par « échantillon biologique » on entend désigner tout prélèvement issu d'un mammifère, notamment humain, ou de liquides biologiques, ou de tissus ou de cellules eucaryotes cultivé(e)s *in vitro* ou *ex vivo,* et susceptibles d'être infecté(e)(s) par un rétrovirus contenant une protéine rétrovirale p24. Par exemple, un échantillon biologique peut être un prélèvement d'un fluide corporel, tel que le sang, le plasma, les extraits leucocytaires, la salive, l'urine, la bile, les larmes, ou le lait maternel, ou encore un surnageant de milieu de culture, un lysat ou un extrait cellulaire ou tissulaire.

Un tissu biologique en culture peut être un tissu reconstitué *in vitro* ou être un tissu prélevé chez un mammifère et cultivé *ex vivo.*

Les cultures de cellules considérées par l'invention peuvent être des cultures de cellules primaires ou des cultures de lignées cellulaires. Elles peuvent être identiques ou de différentes natures, comme par exemple des cellules dendritiques, des leucocytes, des lymphocytes, des monocytes, des macrophages, etc., infectées ou non par le VIH.

Un mammifère considéré par l'invention peut être tout mammifère susceptible d'être infecté par un rétrovirus comprenant une protéine rétrovirale p24, ou un homologue de celle-ci, tel qu'un chat ou un primate, et notamment un être humain.

Une « infection virale » peut être toute infection d'une cellule, d'un tissu ou d'un individu, notamment d'un être humain, par un virus possédant et/ou apte à faire produire par la cellule, le tissu ou l'individu infecté(e), une protéine rétrovirale p24, ou un homologue de celle-ci.

Selon l'invention, par « homologue », on entend désigner, à l'égard d'une première séquence d'acides aminés, toute séquence d'acides aminés présentant au moins 80 %, au moins 85 %, au moins 90 %, au moins 95 %, au moins 98 %, au moins 99 % d'homologie avec cette première séquence d'acides aminés, et présentant une activité biologique similaire.

Une homologie de séquence peut être identifiée par toute technique habituellement utilisée dans le domaine par l'homme de l'art. A titre d'exemple, une homologie de séquence peut être déterminée par utilisation de l'interface informatique BLAST disponible sur le site Internet NCBI à l'adresse http://blast.ncbi.nlm.nih.gov configurée avec les paramètres par défaut.

Un homologue d'une première séquence d'acides aminés peut différer de celle-ci, par exemple, par une ou plusieurs délétion(s), et/ou insertion(s) et/ou une ou plusieurs substitution(s) d'un acide aminé. Ces modifications peuvent être combinées. Un homologue d'une première séquence d'acides aminés peut comprendre une ou plusieurs substitutions conservatrices d'acides aminés. Les modifications présentées ci-dessus peuvent être dénommées, de manière générale, « mutation ». Ainsi, les homologues de l'invention concernent également les mutants et les variants de séquences d'acides aminés de l'invention.

A titre d'exemple de mutations que l'on peut considérer dans la présente invention, on peut mentionner le remplacement d'un ou de plusieurs résidus d'acides aminés par des résidus d'acides aminés ayant un indice hydropathique similaire, sans pour autant affecter de manière sensible les propriétés biologiques de la protéine, et notamment sa propriété d'interaction avec sa protéine partenaire. L'indice hydropathique est un indice attribué aux acides aminés en fonction de leur hydrophobicité et de leur charge (Kyte et Doolittle (1982), J. Mol. Biol., 157: 105).

Par "homologue" au sens de l'invention on entend également couvrir des séquences d'acides aminés issues d'une autre espèce que celle retenue pour la première séquence d'acides aminés, mais possédant le degré d'homologie requis et manifestant les propriétés nécessaires à la mise en oeuvre de l'invention. A titre d'exemple d'homologues considéré par l'invention, on peut citer à l'égard d'un fragment de EF-1α, un fragment de EF-Tu présent chez les procaryotes, et à l'égard de la protéine rétrovirale p24 du VIH-1, les protéines p24 de HTLV-1 et HTLV-2, et la protéine rétrovirale p26 du VIH-2.

Les séquences d'acides aminés convenant à l'invention peuvent être naturelles ou synthétiques, le cas échéant susceptibles d'être obtenues par synthèse chimique ou biologique, ou par extraction à partir d'un tissu biologique, exprimant naturellement ou après transduction, la séquence, ainsi que les différentes formes post-traductionnelles, les homologues, les mutants ou les fragments de celle-ci. Elles peuvent être obtenues par tout procédé connu de l'homme de l'art, notamment comme décrit par SAMBROOK et al. (Molecular Cloning : A laboratory Manual, Ed. Cold Spring Harbor, 2001).

Au sens de l'invention, par « environ » on entend indiquer que la valeur qui suit ce terme est vérifiée en prenant en compte les limites d'erreurs expérimentales acceptables pour l'homme du métier.

Au sens de l'invention, « quantité efficace » signifie la quantité minimale et suffisante pour observer la survenue d'un effet recherché, à savoir par exemple la formation d'un complexe comprenant au moins une protéine rétrovirale p24, ou un homologue de celle-ci, associée à EF-1.

### Protéine facteur d'élongation de la traduction 1 (protéine EF-1)

La protéine Facteur d'Elongation de la traduction 1, ou protéine EF-1, est impliquée dans la phase d'élongation de la synthèse protéique au sein de toutes cellules. Elle intervient dans la liaison GTP-dépendante des aminoacyl-ARNts aux ribosomes. Cette protéine, ubiquitaire, est appelée protéine EF-1 chez les eucaryotes et protéine EF-Tu chez les procaryotes.

La protéine EF-1 plus particulièrement considérée dans la présente invention peut être la protéine EF-1 humaine.

La protéine EF-1 est constituée de quatre sous-unités, à savoir EF-1α, EF-1β, EF-1γ et EF-1δ.

La présente invention peut mettre en oeuvre au moins une sous-unité de EF-1, d'un homologue d'EF-1, ou un fragment C-terminal de ceux-ci.

Selon un mode de réalisation, au moins une sous-unité de la protéine EF-1, voire la protéine EF-1 entière, comprenant les 4 sous-unités mentionnées ci-dessus, peut être mise en oeuvre.

Une sous-unité de la protéine EF-1 convenant à l'invention peut être choisie parmi EF-1α, EF-1β, EF-1γ et EF-1δ. De préférence, une sous-unité mise en oeuvre peut être EF-1α.

Les sous-unités EF-1α, EF-1β, EF-1γ et EF-1δ considérés dans l'invention peuvent être figurées par les séquences identifiées par les références SwissProt respectives P68104, P24534, P26641 et P29692, ou un homologue de celles-ci.

Un homologue de EF-1 convenant à l'invention présente le degré d'homologie précédemment défini, et une activité biologique similaire. Une activité biologique similaire considérée pour un homologue de EF-1 peut être une activité de facteur d'élongation et/ou une aptitude à lier une protéine rétrovirale p24, ou un homologue de celle-ci, et/ou une protéine rétrovirale GAG, ou un homologue de celle-ci. De préférence, un homologue de EF-1 convenant à l'invention possède, le cas échéant à un degré différent, ces trois aptitudes.

Le degré d'homologie conforme à l'invention entre EF-1 et un homologue de celle-ci peut être satisfait sur une portion seulement des séquences d'acides aminés considérées, et de préférence dans la portion C-terminale.

Un fragment C-terminal de EF-1 convenant à l'invention est de longueur suffisante pour être nécessairement doté de la propriété de lier, par exemple par affinité, une protéine rétrovirale p24 ou un homologue de celle-ci. Cette propriété peut être déterminée par toute méthode connue de l'homme de l'art. Par exemple, un fragment C-terminal d'une sous-unité de la protéine EF-1 à tester peut être fixé sur un support, tel qu'une surface plastique. Une protéine rétrovirale p24 est ensuite mis(e) en contact avec le fragment C-terminal, et sa liaison au fragment peut ensuite être détectée au moyen d'un anticorps anti-p24 luminescent.

Un fragment C-terminal d'une sous-unité de la protéine EF-1 convenant à l'invention comprend une séquence d'acides aminés variant de 150 à 250, voire de 160 à 240, et de préférence de 170 à 230 acides aminés contigus de l'extrémité C-terminale de la sous-unité, étant entendu que les acides aminés sont décomptés à partir du dernier acide aminé de la séquence d'acides aminés considérée. Un fragment C-terminal peut comprendre 150, 160, 170, 180, 190, ou 200 acides aminés contigus de l'extrémité C-terminale d'une sous-unité de la protéine EF-1.

Comme indiqué ci-dessus, il est possible d'utiliser un fragment C-terminal de l'une des sous-unités de la protéine EF-1. De préférence, on peut utiliser un fragment C-terminal de la sous-unité EF-1α.

Ainsi, peut convenir à l'invention un homologue d'un fragment C-terminal de EF-1α figuré par un fragment C-terminal de la protéine procaryotique de facteur d'élongation de la traduction EF-Tu.

Peut également convenir à l'invention, la protéine procaryotique de facteur d'élongation de la traduction EF-Tu.

A titre d'exemple de protéines EF-Tu convenant à l'invention, on peut citer les protéines EF-Tu de *Geobacillus stearothermophilus* figuré par la séquence 050306 (Swiss-Prot).

### Polyprotéine virale GAG

Selon un mode de réalisation, EF-1 peut être mise en oeuvre sous une forme associée à une polyprotéine virale GAG, ou un homologue de celle-ci, ledit homologue de GAG présentant au moins 80% d'homologie avec GAG et une activité biologique similaire.

Une telle mise en oeuvre permet avantageusement d'augmenter la sensibilité de capture et de détection de la protéine rétrovirale p24 ou d'un homologue de celle-ci. Egalement, une telle mise en oeuvre permet de diminuer le bruit de fond des procédés d'immunocapture.

La polyprotéine virale GAG est exprimée par les rétrovirus, et notamment par les lentivirus. Cette protéine est clivée durant sa maturation pour donner la protéine de matrice p17, la protéine de capside p24, la protéine de nucléocapside p7, et les protéines p1, p2 et p6. Cette polyprotéine est plus particulièrement décrite par Ganser-Pornillos et al. (Curr. Opin. Struct. Biol., 2008, 18 :203).

De préférence, une polyprotéine virale GAG convenant à l'invention peut être de la même espèce ou souche de virus que la protéine p24, ou l'homologue de celle-ci. Ainsi, une polyprotéine virale GAG convenant à l'invention peut être choisie parmi les polyprotéines virales GAG du VIH, en particulier du VIH-1 ou du VIH-2, ou du HTLV, en particulier du HTLV-1 ou HTLV-2. Plus particulièrement, elle peut-être figurée par une séquence d'acides aminés identifiée par les références SwissProt P04591 ou P18095, ou un homologue de celles-ci.

Un homologue d'une polyprotéine virale GAG convenant à l'invention présente le degré d'homologie précédemment définie et une activité biologique similaire. Une activité biologique d'une polyprotéine GAG à considérer pour l'invention est, de préférence, son aptitude à se lier, dans des conditions appropriées, à EF-1 et à une protéine p24, ou à un homologue de celle-ci. Un homologue d'une polyprotéine virale GAG présent au moins 80 %, au moins 85 %, au moins 90 %, au moins 95 %, au moins 98 %, voire au moins 99 % d'homologie avec les séquences indiquées ci-dessus.

Selon un mode de réalisation, EF-1 et la polyprotéine virale GAG, ou un homologue de celle-ci, peuvent être mis en oeuvre selon un rapport pondéral GAG/EF-1, inférieur ou égal à 1, et de préférence inférieur à 1.

### Protéine rétrovirale détectable

Une protéine rétrovirale détectable au moyen de l'invention est une protéine rétrovirale p24, ou un homologue de celle-ci.

La protéine rétrovirale p24 joue un rôle majeur chez de nombreux lentivirus, et plus particulièrement chez le VIH-1 et les virus HTLV-1 et HTLV-2 (Human T-Lymphotrophic Virus), en constituant la capside renfermant l'information génétique (ARN) du virus. Cette protéine est décrite dans les documents "p24 Antigen Capture Assay for Quantification of Human Immunodeficiency Virus Using Readily Available Inexpensive Reagents", Methods, 1997 Aug ; 12(4) : 288-293, et "Biochemical and Immunological Analysis of Human Immunodeficiency Virus GAG Gene Products p17 and p24", J. Virol., 1998 Mar ; 62(3) : 795-801.

A titre d'exemple de protéines rétrovirales p24 détectables par l'invention on peut citer les protéines figurées par une séquence d'acides aminés identifiée par les références SwissProt P04591 ou P18095, ou un homologue de celles-ci.

A titre d'homologue de la protéine rétrovirale p24, on peut plus particulièrement considérer la protéine rétrovirale p26 du VIH-2.

Une protéine rétrovirale p24, ou un homologue de celle-ci, détectable au moyen de l'invention peut être choisi(e) parmi les protéines rétrovirales p24 du VIH-1, du HTLV-1 ou du HTLV-2 et p26 du VIH-2.

Un virus comprenant une protéine rétrovirale détectable par l'invention peut être choisi parmi le VIH de type 1 ou de type 2, et les virus HTLV-1 et HTLV-2. De préférence, un virus comprenant une protéine rétrovirale détectable par l'invention peut-être le VIH, en particulier le VIH-1.

### Procédé de détection

Un procédé de l'invention pour détecter la présence présumée d'une protéine rétrovirale p24, ou d'un homologue de celle-ci, dans un échantillon biologique repose sur la formation d'un complexe protéique entre EF-1 et une protéine rétrovirale p24, ou l'homologue de celle-ci.

Ainsi, un procédé de l'invention pour détecter, dans un échantillon biologique, la présence présumée d'une protéine rétrovirale p24, ou d'un homologue de celle-ci, comprend au moins les étapes consistant à :
a- mettre en contact ledit échantillon avec une quantité efficace d'au moins une sous-unité d'une protéine facteur d'élongation de la traduction 1, EF-1, d'un homologue de EF-1, ou d'un fragment C-terminal de ceux-ci, dans des conditions appropriées à la formation d'au moins un complexe protéique entre la sous-unité de EF-1, l'homologue de EF-1, ou le fragment C-terminal de ceux-ci, et au moins une protéine rétrovirale p24, ou un homologue de celle-ci,
b- mettre en contact le complexe protéique de l'étape a- avec une entité bi-fonctionnelle, ladite entité étant dotée d'un groupement apte à se lier spécifiquement au moins à une protéine rétrovirale p24, ou un homologue de celle-ci, dans ledit complexe, et d'un groupement apte à être détectable, directement ou indirectement, par un mode de détection, la mise en contact étant effectuée dans des conditions appropriées à l'établissement d'une liaison entre ladite protéine rétrovirale et ladite entité bi-fonctionnelle,
c- détecter ladite entité bi-fonctionnelle, la détection de ladite entité bi-fonctionnelle liée étant indicatrice de la présence de la protéine rétrovirale p24, ou de l'homologue de celle-ci.

Avantageusement, EF-1 peut être immobilisé sur un support comme décrit ci-après.

L'échantillon biologique peut être utilisé brut ou préparé, par exemple par dilution, purification ou ajout de divers réactifs destinés, par exemple, à neutraliser des activités enzymatiques susceptibles d'être délétères pour un procédé de l'invention. L'échantillon biologique est ensuite amené au contact de EF-1 (étape a-), et laissé incubé un temps suffisant pour la formation du complexe protéique EF-1-protéine p24 (ou un homologue de celle-ci). Un temps d'incubation convenant à l'invention peut varier de quelques minutes, par exemple 10, 20, 30 ou 60 minutes, à quelques heures, par exemple 2, 3 ou 4 heures.

Un procédé de l'invention peut prévoir une étape additionnelle consistant à éliminer le surplus d'échantillon biologique, par exemple par rinçage.

Après formation du complexe, une entité bifonctionnelle portant un premier groupe fonctionnel permettant sa liaison avec la protéine p24 (ou l'homologue de celle-ci) dans le complexe protéique et un second groupe fonctionnel permettant, directement ou indirectement, sa détection est amené au contact du complexe protéique (étape b-). Le temps d'incubation de l'entité bifonctionnelle avec le complexe protéique dépend de l'affinité de l'entité envers la protéine p24 (ou un homologue de celle-ci), et peut varier de quelques minutes à quelques heures. Par exemple, le temps d'incubation peut être de 10, 20, 30 ou 60 minutes, ou de 2, 3 ou 4 heures

Un procédé de l'invention peut comprendre, de préférence, au moins une étape supplémentaire après l'étape b- et avant l'étape c-, consistant à éliminer l'entité bifonctionnelle non liée à la protéine virale p24, ou à un homologue de celle-ci, à l'étape b-.

Le complexe protéique EF-1/p24 (ou un homologue de celle-ci) ayant lié l'entité bifonctionnelle est ensuite soumis à une étape de détection convenant à la détection du second groupe fonctionnel (étape c-). Celle-ci peut être effectuée directement, si le second groupe fonctionnel est apte par lui-même à émettre un signal détectable, comme un signal luminescent, radioactif ou coloré, ou indirectement si le second groupe doit être mis à en contact et/ou à réagir avec une ou des entité(s) additionnelle(s), telles que des enzymes, des substrats chromogéniques ou luminescents, ou des anticorps eux-mêmes marqués, pour former un ensemble apte à émettre un signal détectable. Le moyen de détection mis en oeuvre est naturellement adapté à la nature du signal à détecter.

### Immobilisation d'EF-1 sur un support

EF-1 peut être immobilisé sur tout support habituellement utilisé dans le domaine de la fixation et de la détection des protéines. Un support de l'invention peut être tout matériau solide, souple, flexible ou rigide, sur ou dans lequel EF-1 peut être lié(e), adsorbé(e), ou synthétisé(e) *in-situ.*

Un support convenant à l'invention peut être de nature organique ou inorganique, ou encore être une combinaison de matière organique et inorganique. Avantageusement, un support de l'invention peut être constitué d'un matériau de cellulose, de nitrocellulose, en plastique, en verre, en céramique, en silice, ou en résine polymérique.

Un tel support peut se présenter sous toute forme, adaptée à l'invention. Par exemple, un support de l'invention peut être sous la forme de particules, de gels, de feuilles, de tubes, de sphères, par exemples creuses ou microporeuses, de capillaires, de points, de films, de plaques de puits, de bandelettes, ou de colonnes.

En particulier, un support selon l'invention peut être figuré par une plaque de puits, une bille de latex, une bille de verre, une bille de plastique, une bandelette de papier, un microtube, une membrane de nitrocellulose, une membrane de nylon, une puce de silice, une puce recouverte de nanoparticules d'or ou de nanoparticules aimantées, une lame de verre, une surface poreuse, un système microfluidique, ou une nanoparticule aimantée.

De préférence, un support convenant à l'invention peut être une membrane de nitrocellulose ou toute surface convenant à la mise en oeuvre d'un test de type ELISA, telle qu'une plaque de puits.

L'immobilisation de EF-1 sur un support peut être effectuée de manière directe par une ou des liaison(s) covalente(s) ou par adsorption. La méthode d'immobilisation de EF-1 au support est naturellement choisie de sorte à ne pas affecter les propriétés de celle-ci à lier la protéine p24, ou un homologue de celle-ci, et le cas échéant sa capacité à lier une polyprotéine virale GAG (ou un homologue de celle-ci). EF-1 peut être immobilisé sur un support seul ou préalablement associé à la polyprotéine virale GAG (ou à un homologue de celle-ci).

Selon un mode de réalisation préféré, l'immobilisation est effectuée de manière directe par adsorption.

EF-1 peut également être immobilisé sur un support par liaison covalente. Une liaison covalente peut résulter de la réaction d'au moins un des acides aminés de EF-1 ayant une fonction réactive avec une fonction réactive du support. Une fonction réactive portée par un acide aminé peut, par exemple, être une fonction -SH, -OH, ou -COOH. Ces fonctions peuvent, par exemple, conduire à la formation de ponts disulfure par réaction avec une fonction thiol du support, ou de fonction ester ou amide par réaction respectivement avec une fonction acide, alcool, ou aminée du support.

Les fonctions réactives peuvent être portées naturellement par les acides aminés et/ou le support, ou peuvent avoir été introduites sur les acides aminés et/ou le support, par tout processus de modification connu de l'homme de l'art.

Alternativement, EF-1 peut être immobilisé(e) sur un support de façon indirecte, i.e. par l'intermédiaire d'un espaceur.

Le choix d'un espaceur pour la mise en oeuvre de l'invention sera naturellement effectué de sorte à ne pas altérer l'aptitude de EF-1 à lier ou à favoriser la capture de la protéine rétrovirale à détecter.

Selon sa nature, un espaceur convenant à l'invention peut être fixé à EF-1 par réaction chimique, par génération d'une protéine de fusion EF-1-espaceur, ou par affinité. L'espaceur peut être fixé au support par tout type d'interactions ou de liaisons appropriées.

Un espaceur peut être une séquence protéique, pouvant de préférence comprendre de 1 à 200 acides aminés, de préférence de 6 à 100 acides aminés, préférentiellement de 10 à 50 acides aminés. Dans un tel mode de réalisation, la liaison EF-1 - espaceur peut être réalisée par préparation d'une protéine de fusion EF-1 - espaceur ou par une liaison d'affinité entre EF-1 et l'espaceur.

Une protéine de fusion EF-1 - espaceur peut être préparée par production d'une protéine recombinante au moyen de tout outil de biologie moléculaire connu de l'homme de l'art. Une protéine de fusion convenant à l'invention peut être une protéine EF-1 - polyhistidine. Le groupement polyhistidine peut être lié au support par l'intermédiaire d'ions. Egalement, peut convenir à l'invention une protéine de fusion EF-1 avec la streptavidine. Cette protéine de fusion peut être immobilisée sur le support par interaction avec la biotine, préalablement fixée au support, par adsorption ou liaison covalente.

De manière préférée encore, un espaceur convenant à l'invention peut être un anticorps spécifique de EF-1. L'anticorps peut être lié au support par adsorption. A titre d'exemple d'anticorps aptes à lier EF-1 et convenant à titre d'espaceur, on peut citer les anticorps anti-EF-1α sc-68481et sc-68482 (SANTA CRUZ).

Une telle mise en oeuvre peut particulièrement convenir au développement de test sous forme ELISA (Enzyme Linked ImmunoSorbent Assay).

### Entité bifonctionnelle

Une entité bifonctionnelle de l'invention est apte à se lier à au moins une protéine détectable par l'invention dans un complexe tel que défini précédemment.

Une entité bifonctionnelle convenant peut être de préférence un anticorps ou un fragment d'anticorps.

L'entité bifonctionnelle peut être un anticorps polyclonal ou monoclonal, de type IgG, IgA, IgM, ou IgE. Un anticorps convenant à l'invention peut être choisi parmi des anticorps de souris, de rat, de lapin, de chèvre, de cheval, de lama, d'humain ou d'un autre primate.

Peut également convenir à l'invention un fragment d'anticorps ayant les propriétés de liaison définies ci-dessus. Par « fragment d'anticorps », on entend désigner une portion d'un anticorps telle que Fab, Fab', F(ab)₂, F(ab')₂, et d'autres fragments similaires. Ces termes incluent également toute protéine synthétique ou génétiquement modifiée qui peut agir comme un anticorps en se liant à une protéine détectable de l'invention, dans un complexe protéique tel que défini ci-dessus.

Un anticorps ou un fragment d'anticorps convenant à l'invention peut être préparé par tout procédé connu de l'homme du métier dans le domaine, comme décrit, par exemple, dans « Making and using antibodies : a practical handbook » (Howard & Kaser, Ed. CRC, 2006).

Un anticorps convenant à l'invention peut ne pas être spécifique d'un virus donné, mais peut reconnaître une famille de protéines rétrovirales p24 ou homologues de celles-ci appartenant à une famille de virus. Alternativement, un anticorps ou un fragment d'anticorps peut être spécifique d'une protéine détectable par l'invention appartenant à une espèce de virus donné. Par exemple, un anticorps ou un fragment d'anticorps peut être spécifique d'une protéine rétrovirale p24 du VIH, notamment du VIH-1.

Une entité bifonctionnelle conforme à l'invention comprend un groupement apte à lui conférer la propriété d'être détectable, directement ou indirectement, par un mode de détection. Un groupement détectable peut être propice à une détection luminescente, colorimétrique, ou radioactive.

Un groupement détectable « directement » est un groupement apte à générer un signal, par exemple colorimétrique, luminescent, notamment fluorescent, ou radioactif, susceptible d'être détecté directement.

Un groupement luminescent, et en particulier fluorescent, convenant à l'invention peut-être tout marqueur habituellement utilisé dans le domaine par exemple la fluorescéine, le BODIPY, les sondes fluorescentes du type ALEXA, la coumarine et ses dérivés, la phycoerythrine et ses dérivés, ou des protéines fluorescentes telles que la GFP ou le DsRed.

Un groupement radioactif convenant à l'invention peut-être par exemple ³H, ¹²¹I, ¹²³I, le ^{99m}Tc, le ¹⁴C ou ³²P.

Un groupement détectable « indirectement » est un groupement nécessitant la présence d'une ou plusieurs entité(s) additionnelle(s), telles que des enzymes, des substrats chromogéniques ou luminescents, ou des anticorps eux-mêmes marqués, pour former un ensemble apte à émettre un signal détectable.

Un groupement détectable indirectement peut être une portion Fc nue ou portant un marqueur d'affinité, tel qu'un tag polyhistidine, une biotine, ou une streptavidine, ou portant une enzyme, ou un substrat enzymatique apte à générer un signal par hydrolyse. La détection de ce groupement peut être effectuée, respectivement, par mise en oeuvre d'un anticorps secondaire portant un groupement détectable et apte à reconnaître la portion Fc ou le marqueur d'affinité, ou par mise en oeuvre, selon le cas, d'un groupe biotine ou d'un groupe streptavidine portant un groupement détectable, ou par mise en oeuvre d'un substrat spécifique de l'enzyme et apte à émettre un signal détectable par hydrolyse, ou par mise en oeuvre d'une enzyme spécifique du substrat.

Selon un mode de réalisation préférée, un procédé de l'invention peut mettre en oeuvre un anticorps anti-protéine rétrovirale portant un groupement fonctionnel détectable directement ou indirectement, comme défini précédemment.

Avantageusement, un procédé de l'invention comprenant la mise en oeuvre d'une entité bifonctionnelle comprenant un groupe fonctionnel détectable indirectement peut comprendre la mise en oeuvre d'un anticorps portant un substrat enzymatique chromogénique ou une enzyme apte à hydrolyser le substrat enzymatique chromogénique, ou peut comprendre la mise en oeuvre d'un couple anticorps primaire-anticorps secondaire. L'anticorps primaire peut comprendre une portion Fc nue ou portant un marqueur reconnu par l'anticorps secondaire. L'anticorps secondaire peut comprendre un groupe détectable directement comme défini précédemment ou peut comprendre un substrat enzymatique chromogénique ou une enzyme apte à hydrolyser le substrat enzymatique chromogénique.

Selon un mode de réalisation particulier, une enzyme convenant à l'invention peut être une phosphatase alcaline, une tyrosinase, une peroxydase, ou une glucosidase.

Il relève des connaissances de l'homme du métier de déterminer le substrat chromogénique convenant selon l'enzyme considérée.

Lorsque l'enzyme est une phosphatase alcaline, un substrat chromogénique convenant à l'invention peut alors être le BCIP/MBT (5-bromo-4-chloro-3'-indolyphosphate p-toluidine salt - nitro-blue tetrazolium chloride).

Un groupement détectable convenant à l'invention peut être fixé par toutes méthodes appropriées au niveau de l'entité bifonctionnelle. Sa localisation sur l'entité bifonctionnelle est choisie de manière à ne pas affecter l'aptitude de celle-ci à se lier à une protéine rétrovirale p24 (ou à un homologue de celle-ci) dans un complexe protéique de l'invention.

### Mode de détection

Le mode de détection d'une entité bi-fonctionnelle liée à un complexe de l'invention sera naturellement adapté au groupement détectable mis en oeuvre.

Par « mode de détection », on entend tout système capable de détecter l'émission d'un signal par une entité bi-fonctionnelle de l'invention.

Ainsi, un mode de détection de l'invention peut mettre en oeuvre, par exemple, un système de détection en colorimétrie visible, tel que l'oeil nu, un système de détection par radiochimie ou par médecine nucléaire, tel que la scintigraphie, un système de détection par imagerie, par résonance (IRM), par rayons X, par diffusion de lumière, par spectrométrie de masse, par spectroscopie par exemple par fluorescence ou par UV-visible, par ultrasons, par radioactivité, par réfractométrie, optique, piézoélectrique, acoustique, par électrochimie, par conductivité, par pHmétrie ou encore par voie biologique.

Selon un mode de réalisation, un mode de détection plus particulièrement considéré par l'invention s'appuie sur un système de détection en colorimétrie visible, plus particulièrement à l'oeil nu.

La détection peut se faire de manière quantitative ou qualitative.

Une détection qualitative peut conduire à conclure simplement à la présence ou à l'absence de la protéine rétrovirale cherchée. Une détection quantitative peut permettre un dosage de la protéine recherchée dans l'échantillon biologique testé.

La quantification du signal obtenu peut-être effectué par comparaison de son intensité à celui obtenu avec un échantillon contrôle dénué de la protéine rétrovirale recherchée, et, le cas échéant, à celui obtenu avec un échantillon témoin comprenant la protéine rétrovirale cherchée à une concentration connue.

De manière préférée, il est possible de réaliser une série d'échantillons témoins de concentrations connues, et croissantes, de la protéine rétrovirale cherchée de sorte à établir une courbe étalon à l'égard de laquelle l'intensité du signal mesuré dans l'échantillon à tester sera comparée. Les valeurs de référence de l'échantillon contrôle et des échantillons témoins peuvent être déterminées simultanément à la mesure de l'échantillon test ou être déterminées antérieurement ou postérieurement. Lorsque les valeurs de référence sont déterminées antérieurement, celles-ci peuvent être stockées dans une base de données et être utilisées pour une série de mesures d'échantillons tests.

### Kit

La présente invention concerne également un kit de détection d'au moins une protéine rétrovirale p24, ou un homologue de celle-ci, comprenant:
- au moins un premier réceptacle comprenant au moins une sous-unité d'une protéine facteur d'élongation de la traduction 1, EF-1, un homologue de EF-1, ou un fragment C-terminal de ceux-ci, et
- au moins un second réceptacle comprenant une entité bi-fonctionnelle dotée d'un groupement apte à se lier spécifiquement au moins à ladite protéine rétrovirale p24, ou ledit homologue de celle-ci, susceptible d'être présent(e) dans un complexe protéique formé avec la sous-unité de EF-1, l'homologue de EF-1, ou le fragment C-terminal de ceux-ci, et un groupement apte à être détectable, directement ou indirectement, par un mode de détection.

Selon un mode de réalisation, un kit de l'invention peut en outre comprendre une polyprotéine virale GAG ou un homologue de celle-ci.

La polyprotéine virale GAG ou l'homologue de celle-ci peut être disposé(e) dans un réceptacle supplémentaire ou dans le même réceptacle que celui comprenant EF-1.

Un kit de l'invention peut en outre comprendre au moins un réceptacle comprenant une protéine p24, ou un homologue de celle-ci, en quantité ou concentration connue. Ce mode de réalisation peut permettre la réalisation d'un contrôle positif ou d'une courbe étalon en protéine p24 (ou homologue de celle-ci).

Un kit de l'invention peut comprendre en outre au moins une instruction de mise en oeuvre de EF-1, le cas échéant associée à GAG (ou à un homologue de celle-ci), et de l'entité bi-fonctionnelle pour détecter la présence présumée d'une protéine rétrovirale p24 (ou d'un homologue de celle-ci) dans un échantillon biologique.

EF-1, GAG, l'entité bifonctionnelle, p24, et leurs homologues sont avantageusement tels que définis précédemment.

Selon un aspect de l'invention, EF-1 peut être utilisé comme agent de capture d'une protéine rétrovirale p24 ou de l'un de ses homologues. Cet aspect de l'invention peut être mis en oeuvre à des fins de purification d'un échantillon biologique, ou d'enrichissement d'un échantillon en protéine p24.

Dans une telle mise en oeuvre, l'utilisation d'une entité bifonctionnelle telle que définie précédemment n'est pas nécessaire.

Après formation d'un complexe protéique EF-1/p24 (ou homologue de celle-ci), une étape de précipitation ou de séparation du complexe obtenu peut être effectuée, par tout moyen connu de l'homme de l'art. Le complexe est ensuite dissocié pour récupérer la protéine rétrovirale. L'étape de dissociation peut-être effectuée par toute méthode connue dans le domaine, tel quel l'utilisation d'un tampon salin adéquat.

Avantageusement, ce mode de réalisation peut être effectué par mise en oeuvre d'une colonne de chromatographie fonctionnalisée au moyen d'EF-1.

Dans la description ci-avant, l'expression « compris entre » ou « allant de » doit s'entendre bornes incluses.

Les exemples ci-après sont donnés uniquement à titre illustratif de l'invention.

### LEGENDES DES FIGURES

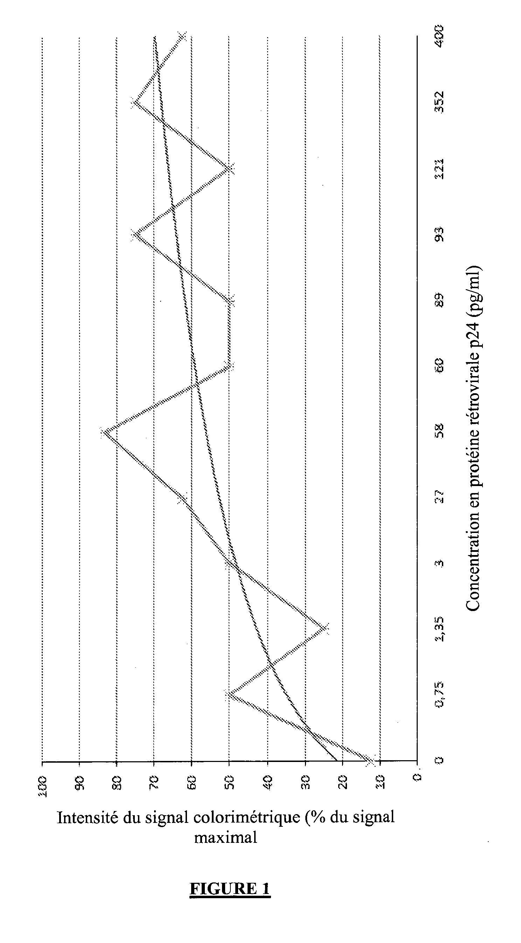

**Figure 1** **:** illustre les variations de l'intensité du signal colorimétrique, en % du signal maximal obtenu par hydrolyse du substrat BCIP-NBT par la phosphatase alcaline de l'anticorps secondaire fixé à l'anticorps primaire de souris anti-protéine rétrovirale p24 lié au complexe EF-1α / protéine p24, en fonction de la concentration de celle-ci. La courbe lisse représente la transformation logarithmique des résultats.

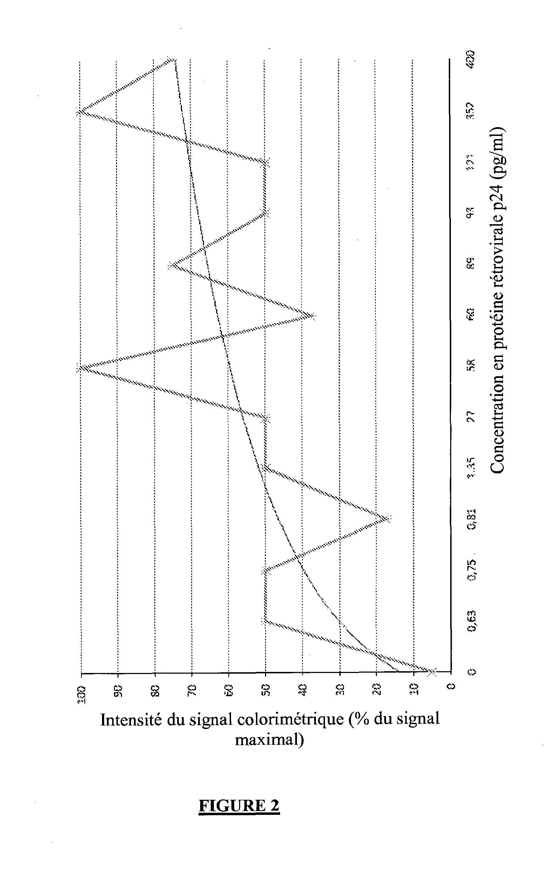

**Figure 2****:** illustre les variations de l'intensité du signal colorimétrique, en % du signal maximal résultant de l'hydrolyse du substrat BCIP-NBT par la phosphatase alcaline de l'anticorps secondaire fixé à l'anticorps primaire de souris anti-p24 fixé au complexe EF-1α / GAG / p24 en fonction de la concentration en protéine p24. EF-1α et GAG sont mis en oeuvre, respectivement, à raison de 100 ng/µL et 50 ng/µL. La courbe lisse représente la transformation logarithmique des résultats obtenus.

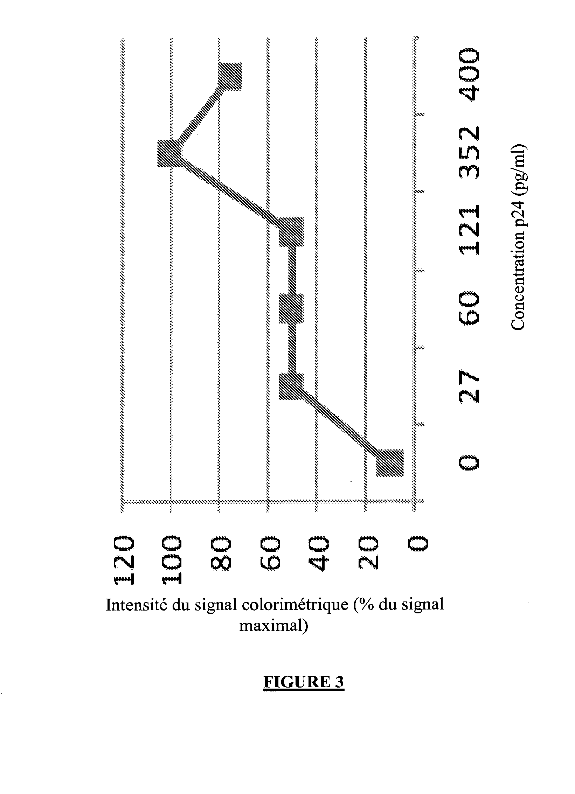

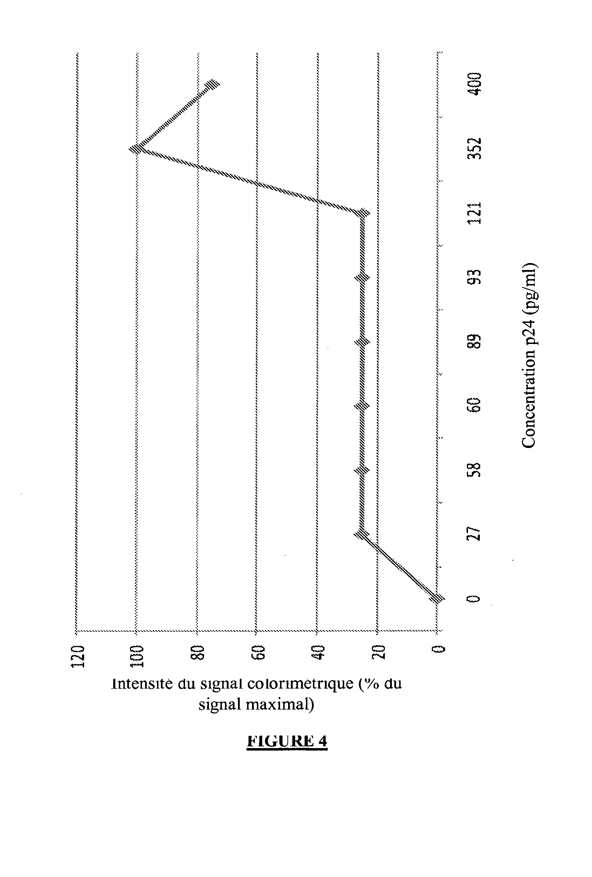

**Figures 3** **et** **4** **:** illustrent la variation de l'intensité du signal colorimétrique en pourcentage du signal maximal obtenu lors de l'expérience, résultant de l'hydrolyse de BCIP-NBT par la phosphatase alcaline d'un anticorps secondaires fixé à l'anticorps primaire de souris anti-p24, lui-même fixé au complexe EF-1α / GAG / p24 en fonction de la concentration en protéine rétrovirale p24 et en fonction de la concentration en polyprotéine virale GAG mise en oeuvre à raison de 25 ng/µL (Figure 3) et 100 ng/µL (Figure 4). La concentration en EF-1α mise en oeuvre est constante, à 100 ng/µL.

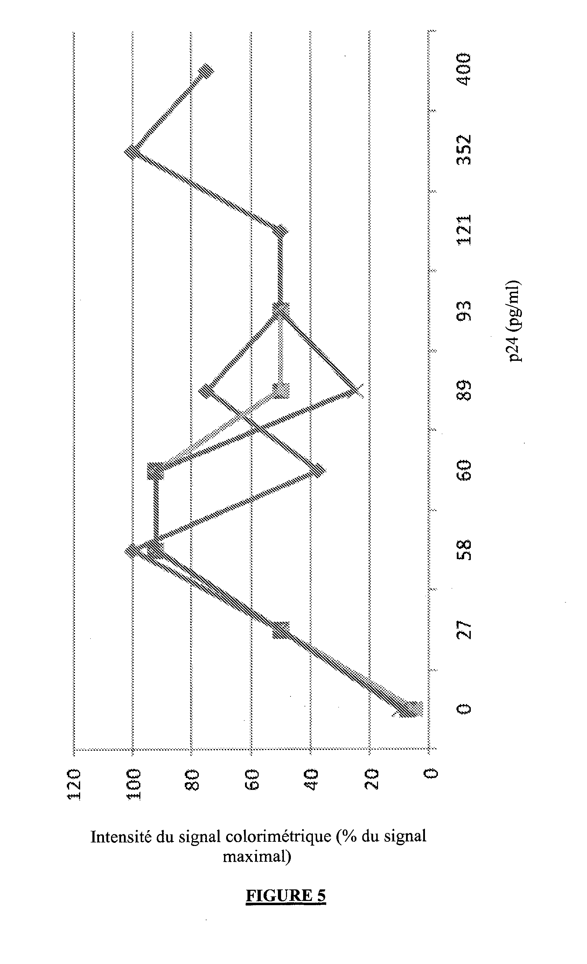

**Figure 5****:** illustre la variation du signal colorimétrique, en pourcentage du signal maximal obtenu, par hydrolyse des substrats BCIP-NBT par la phosphatase alcaline de l'anticorps secondaire fixé à l'anticorps primaire anti-p24 lui-même fixé au complexe EF-1α / GAG / p24 en fonction de la concentration en protéine p24 et de la nature de l'anticorps primaire utilisé. EF-1α et GAG sont mis en oeuvre, respectivement, à raison de 100 ng/µL et 50 ng/µL. Les anticorps primaires de souris utilisés sont, respectivement, sc-69 728 (losange), sc-65 462 (carré), sc-57 827 (triangle) et sc-73 300 (croix), tous de SANTA CRUZ BIOTECHNOLOGY. L'anticorps secondaire est un anticorps de chèvre anti-anticorps de souris marqué à l'alcaline phosphatase dilué (SANTA CRUZ BIOTECHNOLOGY sc.-2008).

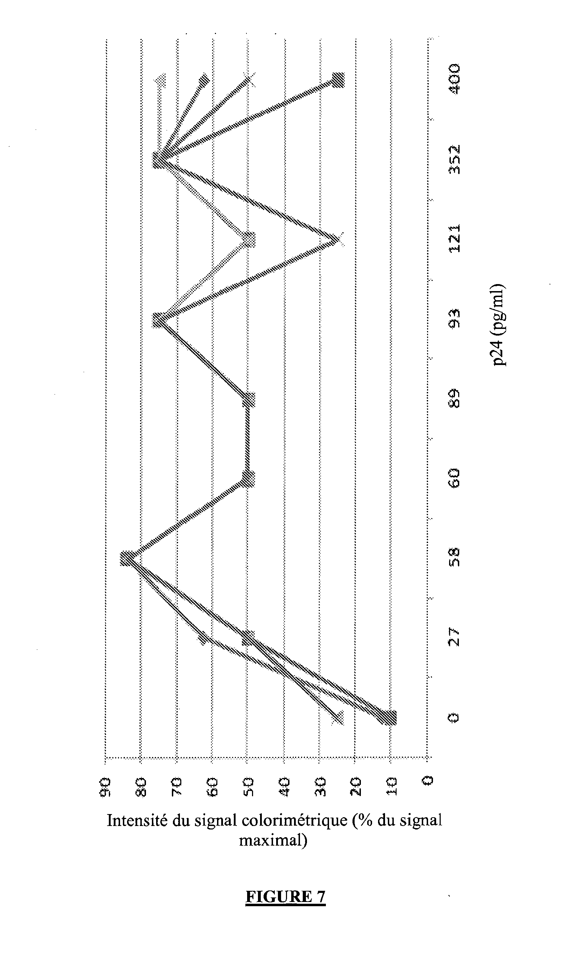

**Figures 7** et 8 **:** illustrent la variation de l'intensité du signal colorimétrique, en pourcentage du signal maximal, après hydrolyse du substrat BCIP-NBT par la phosphatase alcaline de l'anticorps secondaires fixé à l'anticorps primaire anti-p24, lui-même fixé au complexe EF-1α / GAG / p24 en fonction de la concentration en protéine p24, et selon la nature, d'une part, de l'anticorps primaire, et, d'autre part, de l'anticorps secondaire. Les anticorps primaires de souris utilisés sont, respectivement, sc-69728 (losange), sc-65462 (carré), sc-57 827 (triangle), sc-73300 (croix), tous de SANTA CRUZ BIOTECHNOLOGY. Les anticorps secondaires de chèvre anti-souris testés sont respectivement S3721 de PROMEGA (Figure 7) et sc-15065 de SANTA CRUZ BIOTECHNOLOGY (Figure 8).

### EXEMPLES

### Exemple 1

### Interaction entre une sous-unité EP-1α et une protéine rétrovirale p24

La sous-unité EF-1α de la protéine EF-1 humaine est adsorbée à la surface d'une membrane de nitrocellulose par dépôt d'1 µL d'une solution comprenant 100 ng/µL de protéine EF-1α humaine (PROSPEC référence PRO-773). Les dépôts, ou spots, sont effectués en ligne sur la membrane et espacés chacun d'environ 1 cm.

La membrane est laissée sécher, et les sites de liaison non spécifiques de la membrane sont saturés par incubation pendant 1 heure dans une solution de saturation (5 % de lait écrémé dans du Tris Buffer Saline-2,5 % Tween 20 (TBS-T), vendu par la société Sigma-Aldrich), à température ambiante.

Des échantillons (1 uL) de plasma ou de sérum humain comprenant chacun une concentration donnée de protéine rétrovirale p24 (FITZGERALD INDUSTRIES) à, respectivement, 0,75 pg/ml, 1,35 pg/ml, 3 pg/ml, 27 pg/ml, 58 pg/ml, 60 pg/ml, 89 pg/ml, 93 pg/ml, 121 pg/ml, 352 pg/ml, et 400 pg/ml sont déposés sur la membrane de nitrocellulose. Chaque échantillon est déposé sur un spot différent.

La membrane est rincée puis est mise à incuber dans une solution d'anticorps primaire de souris anti-p24 VIH-I dilué au 1/50^{ème} (SANTA CRUZ BIOTECHNOLOGY, sc.-69728).

La membrane est rincée dans du TBS-T puis mise à incuber pendant 30 minutes à température ambiante dans une solution d'anticorps secondaire de chèvre anti-anticorps de souris marqué à l'alcaline phosphatase dilué au 1/2500^{ème} (SANTACRUZ BIOTECHNOLOGY sc.-2008).

La membrane est à nouveau rincée dans une solution de TBS-T, puis dans une solution de TBS (Tris Buffer Saline).

La membrane est ensuite laissée à incuber dans une solution de BCIP/NBT (5-bromo-4-chloro-3'-indolyphosphate p-toluidine salt-nitro-blue tetrazolium chloride) (Sigma-Aldrich) jusqu'à apparition de la coloration environ 5 à 15 minutes.

Enfin, la membrane est rincée à l'eau distillée puis l'intensité de la coloration des différents spots est lue qualitativement à l'oeil nue.

Les résultats sont exprimés en pourcentage d'intensité par rapport au signal maximum obtenu dans l'expérience. Une valeur correspondant à 50 % du signal maximal et retenu comme indiquant positivement la présence de la protéine virale p24.

Les résultats obtenus présentés Figure 1 montrent, d'une part, que la sous-unité EF-1α de la protéine EF-1, et la protéine rétrovirale p24 interagissent l'une avec l'autre. Le complexe formé peut être détectée avec une bonne sensibilité, à de très faibles concentrations en protéine rétrovirale p24 (50 % du signal maximal à environ 3 pg/ml). Cette bonne sensibilité de détection permet avantageusement de diagnostiquer de façon précoce une infection chez un individu par un virus exprimant une protéine rétrovirale de type p24 tel que le VIH-1 (ou p26 chez le VIH-2).

### Exemple 2

### Interaction entre une sous-unité EF-1α / polyprotéine GAG et une protéine rétrovirale p24

Le protocole mis en place dans cet exemple est similaire à celui de l'Exemple 1, à ceci près qu'il comprend une étape supplémentaire de dépôt sur les spots de EF-1α d'une solution de polyprotéine virale GAG (50 ng/µL ; Millipore).

Les anticorps primaires et secondaires utilisés sont les mêmes que ceux de l'Exemple 1 et sont mis en oeuvre à la même concentration.

La révélation du signal colorimétrique se fait également comme indiqué à l'Exemple 1.

Les résultats, présentés en Figure 2, montrent que la formation d'un complexe sous unité EF-1α / polyprotéine GAG favorise la liaison de la protéine p24, permettant une augmentation significative de la sensibilité de la détection de cette protéine.

En effet, 50 % du signal maximal est cette fois obtenu dès 1,10 pg/ml de protéine rétrovirale p24.

Une telle concentration est représentative d'un stade très précoce d'infection par un virus, qui n'est en général pas détectable par les autres tests de diagnostic actuellement disponible, notamment ceux s'appuyant sur la détection de la p24, et potentiellement plus précoce que ceux basés sur la détection de la présence d'ADN ou d'ARN viral.

### Exemple 3

### Effet du ratio sous-unité EF-1α l polyprotéine virale GAG sur la détection d'une protéine rétrovirale p24.

Le procédé mis en oeuvre est similaire à celui de l'Exemple 2.

La protéine GAG a été testée à la concentration, respectivement, de 25 ng/µL (Figure 3) et 100 ng/µL (Figure 4), tout en conservant constante la concentration en sous-unités EF-1α (100 ng/µl). Comme le montre la comparaison des figures 2 (GAG 50 ng/µL), 3 (GAG 25 ng/µL) et 4 (GAG 100 ng/µL) un rapport pondéral GAG/EF-1α inférieur à 1 conduit au meilleur résultat, et permet la détection de la protéine rétrovirale p24 à de très faibles concentrations.

En revanche, pour un rapport pondéral GAG / EF-1α égal à 1, la sensibilité de détection est réduite (seuil de 50 % du signal colorimétrique atteint pour les concentrations supérieures à 121 pg/ml). Toutefois, ce seuil de détection demeure convenable pour la mise en oeuvre d'un test diagnostic.

### Exemple 4

### Effet de la variation de la nature de l'anticorps primaire sur la détection d'une protéine rétrovirale p24 au moyen du complexe sous unités EF-Iα / polyprotéine virale GAG.

Le procédé mis en oeuvre est similaire à celui de l'Exemple 1.

Les anticorps testés sont des anticorps monoclonaux de souris anti-protéine p24 du VIH-1, sc-69728, sc-65462, sc-57827, sc-73300 (SANTA CRUZ BIOTECHNOLOGY). Les concentrations utilisées sont celles de l'Exemple 1. L'anticorps secondaire est également celui de l'Exemple 1.

Les résultats illustrés par la Figure 5 montrent une excellente reproductibilité de la détection et du seuil de détection de la protéine p24, quelque soit la nature de l'anticorps primaire utilisé sc-69728 (losange), sc-65162 (carré), sc-57 827 (triangle), ou sc-73300 (croix). Ces résultats révèlent la robustesse du test diagnostic de l'invention.

### Exemple 5

### Effet de la variation de la nature de l'anticorps secondaire sur la détection d'une protéine rétrovirale p24 au moyen du complexe EF-1α / polyprotéine virale GAG.

Le procédé mis en oeuvre est similaire à celui des Exemples 1 et 4.

Les anticorps primaires de souris utilisés sont, respectivement, sc-69728 (losange), sc-65462 (carré), sc-57827 (triangle) et sc-73300 (croix), tous de SANTA CRUZ BIOTECHNOLOGY.

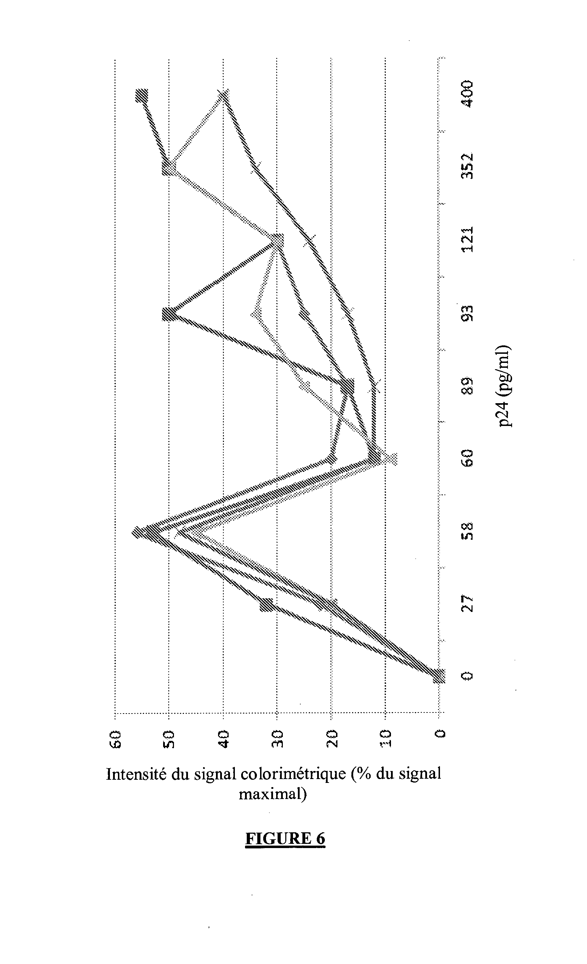

Deux anticorps secondaires de chèvre, couplées à la phosphatase alcaline, anti-anticorps de souris ont été utilisés, à savoir S 3721 (PROMEGA ; Figure 6) et sc-15065 (SANTA CRUZ BIOTECHNOLOGY; Figure 7). Ces anticorps ont été utilisés comme indiqué aux exemples précèdent à savoir dilués au 1/2500^{ème}.

La comparaison des Figures 7 et 8 montrent que la nature de l'anticorps secondaire n'affecte pas sensiblement la détection de la protéine p24, ni le seuil de détection, réalisé au moyen des différents anticorps primaires précédemment testés.

Ces résultats démontrent encore la robustesse et la reproductibilité du test de détection de la protéine rétrovirale p24, ainsi que de ses homologues tel que la protéine rétrovirale p26 du VIH-2.

## Revendications

1. Utilisation, *in vitro* ou *ex vivo,* d'au moins une sous-unité α d'une protéine Facteur d'Elongation de la traduction 1, EF-1, d'un homologue de EF-1, ledit homologue de EF-1 présentant au moins 80% d'homologie avec EF-1 et une activité biologique similaire ou d'un fragment C-terminal de ceux-ci comprenant une séquence d'acides aminés variant de 150 à 250 acides aminés contigus de l'extrémité C-terminale de la sous-unité ou de son homologue, comme agent de détection d'une protéine rétrovirale p24, ou d'un homologue de celle-ci, ledit homologue de p24 présentant au moins 80% d'homologie avec p24 et une activité biologique similaire.

2. Utilisation selon la revendication précédente, dans laquelle la sous-unité de EF-1, l'homologue de EF-1, ou le fragment C-terminal de ceux-ci, tels que définis en revendication 1, est mis(e) en oeuvre sous une forme associée à une polyprotéine virale GAG, ou un homologue de celle-ci, ledit homologue de GAG présentant au moins 80% d'homologie avec GAG et une activité biologique similaire.

3. Utilisation selon la revendication 1 ou 2, dans laquelle EF-1 est une protéine EF-1 humaine.

4. Utilisation selon l'une quelconque des revendications précédentes, dans laquelle la protéine rétrovirale p24, ou un homologue de celle-ci tel que défini en revendication 1, est choisi(e) parmi les protéines rétrovirales p24 du VIH-1, du HTLV-1 ou du HTLV-2 et p26 du VIH-2.

5. Procédé, *in vitro* ou *ex vivo,* pour détecter la présence présumée d'une protéine rétrovirale p24, ou d'un homologue de celle-ci, ledit homologue de p24 présentant au moins 80% d'homologie avec p24 et une activité biologique similaire, dans un échantillon biologique, ledit procédé comprenant au moins les étapes consistant à :
a- mettre en contact ledit échantillon avec une quantité efficace d'au moins une sous-unité α d'une protéine facteur d'élongation de la traduction 1, EF-1, d'un homologue de EF-1, ledit homologue de EF-1 présentant au moins 80% d'homologie avec EF-1 et une activité biologique similaire, ou d'un fragment C-terminal de ceux-ci comprenant une séquence d'acides aminés variant de 150 à 250 acides aminés contigus de l'extrémité C-terminale de la sous-unité ou de son homologue, dans des conditions appropriées à la formation d'au moins un complexe protéique entre la sous-unité de EF-1, l'homologue de EF-1, ou le fragment C-terminal de ceux-ci, et au moins une protéine rétrovirale p24, ou l'homologue de celle-ci,
b- mettre en contact le complexe protéique de l'étape a- avec une entité bi-fonctionnelle, ladite entité étant dotée d'un groupement apte à se lier spécifiquement au moins à une protéine rétrovirale p24, ou l'homologue de celle-ci, dans ledit complexe, et d'un groupement apte à être détectable, directement ou indirectement, par un mode de détection, la mise en contact étant effectuée dans des conditions appropriées à l'établissement d'une liaison entre ladite protéine rétrovirale et ladite entité bi-fonctionnelle.
c- détecter ladite entité bi-fonctionnelle, la détection de ladite entité bi-fonctionnelle liée étant indicatrice de la présence de la protéine rétrovirale p24, ou de l'homologue de celle-ci.

6. Procédé selon la revendication précédente, comprenant au moins une étape supplémentaire après l'étape b- et avant l'étape c-, ladite étape supplémentaire consistant à éliminer l'entité bi-fonctionnelle non liée à la protéine virale p24, ou à l'homologue de celle-ci tel que défini en revendication 5, à l'étape b-.

7. Procédé selon la revendication précédente, dans lequel la sous-unité de EF-1, l'homologue de EF-1, ou le fragment C-terminal de ceux-ci, tels que définis en revendication 5, est immobilisé(e) sur un support organique ou inorganique.

8. Procédé selon la revendication précédente, dans lequel le support est constitué d'un matériau de cellulose, de nitrocellulose, en plastique, en verre, en céramique, en silice, ou en résine polymérique.

9. Procédé selon l'une quelconque des revendications 5 à 8, dans lequel ladite entité bi-fonctionnelle est un anticorps ou un fragment d'anticorps.

10. Procédé selon l'une quelconque des revendications 5 à 9, dans lequel ledit groupement détectable est un groupement propice à une détection luminescente, colorimétrique, ou radioactive.

11. Procédé selon l'une quelconque des revendications 5 à 10, dans lequel la sous-unité de EF-1, l'homologue de EF-1 tel que défini en revendication 5, ou le fragment C-terminal de ceux-ci tel que défini en revendication 5 est mis(e) en oeuvre sous une forme associée à une polyprotéine virale GAG, ou un de homologue de celle-ci, ledit homologue de GAG présentant au moins 80% d'homologie avec GAG et une activité biologique similaire.

12. Kit de détection d'au moins une protéine rétrovirale p24, ou un homologue de celle-ci, ledit homologue de p24 présentant au moins 80% d'homologie avec p24 et une activité biologique similaire, comprenant :
- au moins un premier réceptacle comprenant au moins une sous-unité α d'une protéine facteur d'élongation de la traduction 1, EF-1, un homologue de EF-1, ledit homologue de EF-1 présentant au moins 80% d'homologie avec EF-1 et une activité biologique similaire, ou un fragment C-terminal de ceux-ci comprenant une séquence d'acides aminés variant de 150 à 250 acides aminés contigus de l'extrémité C-terminale de la sous-unité ou de son homologue, et
- au moins un second réceptacle comprenant une entité bi-fonctionnelle dotée d'un groupement apte à se lier spécifiquement au moins à ladite protéine rétrovirale p24, ou ledit homologue de celle-ci, susceptible d'être présent(e) dans un complexe protéique formé avec la sous-unité de EF-1, l'homologue de EF-1, ou le fragment C-terminal de ceux-ci, et un groupement apte à être détectable, directement ou indirectement, par un mode de détection.

13. Kit selon la revendication précédente, comprenant en outre une polyprotéine virale GAG ou un homologue de celle-ci, ledit homologue de GAG présentant au moins 80% d'homologie avec GAG et une activité biologique similaire.

## Patentansprüche

1. Verwendung, *in vitro* oder *ex vivo,* von mindestens einer α-Untereinheit eines Translationsverlängerungsfaktor-1-, EF-1-Proteins, eines Homologen von EF-1, wobei das Homolog von EF-1 mindestens 80 % Homologie mit EF-1 und eine ähnliche biologische Aktivität aufweist, oder eines C-terminalen Fragments davon, das eine Sequenz von Aminosäuren von 150 bis 250 zusammenhängenden Aminosäuren des C-terminalen Endes der Untereinheit oder ihres Homologen umfasst, als Nachweismittel eines retroviralen p24-Proteins oder eines Homologen davon, wobei das Homolog von p24 mindestens 80 % Homologie mit p24 und eine ähnliche biologische Aktivität aufweist.

2. Verwendung nach dem vorhergehenden Anspruch, wobei die EF-1-Untereinheit, das EF-1-Homolog, oder das C-terminale Fragment davon, wie in Anspruch 1 definiert, in einer Form bereitgestellt wird, die mit einem viralen GAG-Polyprotein oder einem Homolog davon verknüpft ist, wobei das Homolog von GAG mindestens 80 % Homologie mit GAG und eine ähnliche biologische Aktivität aufweist.

3. Verwendung nach dem Anspruch 1 oder 2, wobei EF-1 ein menschliches EF-1-Protein ist.

4. Verwendung nach einem der vorhergehenden Ansprüche, wobei das retrovirale p24-Protein oder ein Homolog davon, wie in Anspruch 1 definiert, aus retroviralen p24-Proteinen von VIH-1, HTLV-1 oder HTLV-2 und p26 von VIH-2 ausgewählt ist.

5. Verfahren, *in vitro* oder *ex vivo,* zum Nachweisen der vermuteten Gegenwart eines retroviralen p24-Proteins oder eines Homologen davon, wobei das Homolog von p24 mindestens 80 % Homologie mit p24 und eine ähnliche biologische Aktivität aufweist, in einer biologischen Probe, wobei das Verfahren mindestens die folgenden Schritte umfasst:
a - in Kontakt bringen der Probe mit einer wirksamen Menge von mindestens einer α-Untereinheit eines Translationsverlängerungsfaktor-1-, EF-1-Proteins, eines Homologen von EF-1, wobei das Homolog mindestens 80 % Homologie mit EF-1 und eine ähnliche biologische Aktivität aufweist, oder eines C-terminalen Fragments davon, das eine Sequenz von Aminosäuren von 150 bis 250 zusammenhängenden Aminosäuren des C-terminalen Endes der Untereinheit oder ihres Homologen umfasst, unter Bedingungen, die zur Bildung von mindestens einem Proteinkomplex zwischen der Untereinheit von EF-1, dem Homolog von EF-1 oder dem C-terminalen Fragment davon und mindestens einem retroviralen p24-Protein oder einem Homologen davon geeignet sind,
b - in Kontakt bringen des Proteinkomplexes aus Schritt a mit einer biofunktionelle Einheit, wobei die Einheit mit einer Gruppe ausgestattet ist, die dazu ausgelegt ist, spezifisch mindestens an ein retrovirales p24-Protein oder das Homolog davon in dem Komplex zu binden, und einer Gruppe, die dazu ausgelegt ist, direkt oder indirekt durch eine Nachweisart nachweisbar zu sein, wobei das Inkontaktbringen unter Bedingungen durchgeführt wird, die zur Entwicklung einer Bindung zwischen dem retroviralen Protein und der bifunktionellen Einheit geeignet sind,
c - Nachweisen der bifunktionellen Einheit, wobei der Nachweis der gebundenen bifunktionellen Einheit ein Hinweis auf die Gegenwart des retroviralen p24-Proteins oder des Homologen davon ist.

6. Verfahren nach dem vorhergehenden Anspruch, umfassend mindestens einen zusätzlichen Schritt nach dem Schritt b und vor dem Schritt c, wobei der zusätzliche Schritt aus dem Eleminieren der nicht an das virale p24-Protein oder an das Homolog davon gebundenen bifunktionellen Einheit, wie in Anspruch 5 in Schritt b definiert, besteht.

7. Verfahren nach dem vorhergehenden Anspruch, wobei die Untereinheit von EF-1, das Homolog von EF-1 oder das C-terminale Fragment davon, wie in Anspruch 5 definiert, auf einem organischen oder anorganischen Träger immobilisiert wird.

8. Verfahren nach dem vorhergehenden Anspruch, wobei der Träger aus einem Cellulosematerial, Nitrocellulosematerial, aus Kunststoff, aus Glas, aus Keramik, aus Siliciumdioxid oder aus polymerem Harz besteht.

9. Verfahren nach einem der Ansprüche 5 bis 8, wobei die bifunktionelle Einheit ein Antikörper oder ein Antikörperfragment ist.

10. Verfahren nach einem der Ansprüche 5 bis 9, wobei die nachweisbare Gruppe eine Gruppe ist, die für einen Lumineszenz-Nachweis, einen coloumetrischen Nachweis oder einen radioaktiven Nachweis günstig ist.

11. Verfahren nach einem der Ansprüche 5 bis 10, wobei die Untereinheit von EF-1, das Homolog von EF-1, wie in Anspruch 5 definiert, oder das C-terminale Fragment davon, wie in Anspruch 5 definiert, in einer Form bereitgestellt wird, die mit einem viralen GAG-Polyprotein oder einem Homolog davon verknüpft ist, wobei das Homolog von GAG mindestens 80 % Homologie mit GAG und eine ähnliche biologische Aktivität aufweist.

12. Nachweiskit von mindestens einem retroviralen p24-Protein oder einem Homologen davon, wobei das Homolog von p24 mindestens 80 % Homologie mit p24 und eine ähnliche biologische Aktivität aufweist, umfassend:
- mindestens ein erstes Gefäß umfassend mindestens eine α-Untereinheit eines Translationsverlängerungsfaktor-1-, EF-1-Proteins, ein Homolog von EF-1, wobei das Homolog von EF-1 mindestens 80 % Homologie mit EF-1 und eine ähnliche biologische Aktivität aufweist, oder ein C-terminales Fragment davon, das eine Sequenz von Aminosäuren von 150 bis 250 zusammenhängenden Aminosäuren des C-terminalen Endes der Untereinheit oder ihres Homologen umfasst, und
- mindestens ein zweites Gefäß umfassend ein bifunktionelle Einheit, die mit einer Gruppe ausgestattet ist, die dazu ausgelegt ist, spezifisch mindestens an das retrovirale p24-Protein oder das Homolog davon zu binden, die dazu geeignet ist, in einem Proteinkomplex vorhanden zu sein, der mit der Untereinheit von EF-1, dem Homolog von EF-1 oder dem C-terminalen Fragment davon gebildet ist, und eine Gruppe, die dazu ausgelegt ist, direkt oder indirekt durch eine Nachweisart nachweisbar zu sein.

13. Kit nach dem vorhergehenden Anspruch, weiterhin umfassend ein virales GAG-Polyprotein oder ein Homolog davon, wobei das Homolog von GAG mindestens 80 % Homologie mit GAG und eine ähnliche biologische Aktivität aufweist.

## Claims

1. Use *in vitro* or *ex vivo* of at least one subunit α of a translational elongation factor 1 protein, EF-1, a homologue of EF-1, said homologue of EF-1 having at least 80% homology with EF-1 and a similar biological activity, or a C-terminal fragment thereof comprising a sequence of amino acids varying from 150 to 250 amino acids contiguous of the C-terminal end of the subunit or its homologue, as a detection agent for a p24 retroviral protein or a homologue thereof, said homologue of p24 having at least 80% homology with p24 and a similar biological activity.

2. Use according to the preceding claim, wherein the subunit of EF-1, the homologue of EF-1, or the C-terminal fragment thereof as defined in claim 1, is implemented in a form associated with a GAG viral polyprotein or a homologue thereof, said homologue of GAG having at least 80% homology with GAG and a similar biological activity.

3. Use according to claim 1 or 2, wherein EF-1 is a human EF-1 protein.

4. Use according to any of the preceding claims, wherein the p24 retroviral protein or a homologue thereof as defined in claim 1 is selected from the p24 retroviral proteins of HIV-1, HTLV-1 or HTLV-2 and p26 of HIV-2.

5. Method *in vitro* or *ex vivo* for detecting the presumed presence of a p24 retroviral protein or a homologue thereof, said homologue of p24 having at least 80% homology with p24 and a similar biological activity, in a biological specimen, said method comprising at least the steps consisting of:
a) bringing said specimen into contact with an effective quantity of at least one subunit α of a translational elongation factor 1 protein, EF-1, a homologue of EF-1, said homologue of EF-1 having at least 80% homology with EF-1 and a similar biological activity, or a C-terminal fragment thereof comprising a sequence of amino acids varying from 150 to 250 amino acids contiguous of the C-terminal end of the subunit or its homologue, under conditions appropriate for the formation of at least one proteic complex between the subunit of EF-1, the homologue of EF-1 or the C-terminal fragment thereof, and at least one p24 retroviral protein or the homologue thereof,
b) bringing the proteic complex of step a) into contact with a bifunctional entity, said entity being provided with a grouping able to bond specifically to at least one p24 retroviral protein or a homologue thereof in said complex, and with a grouping suitable for being detectable directly or indirectly by a detection mode, the contact being created under conditions appropriate for establishing a bond between said retroviral protein and said bifunctional entity,
c) detecting said bifunctional entity, the detection of said bonded bifunctional entity being indicative of the presence of the p24 retroviral protein or the homologue thereof.

6. Method according to the preceding claim, comprising at least one additional step after step b) and before step c), said additional step comprising eliminating the bifunctional entity not bonded to the p24 viral protein or the homologue thereof as defined in claim 5 in step b).

7. Method according to the preceding claim, wherein the subunit of EF-1, the homologue of EF-1 or the C-terminal fragment thereof as defined in claim 5, is immobilised on an organic or inorganic support.

8. Method according to the preceding claim, wherein the support comprises a material of cellulose, nitrocellulose, plastic, glass, ceramic, silica or polymer resin.

9. Method according to any of claims 5 to 8, wherein said bifunctional entity is an antibody or an antibody fragment.

10. Method according to any of claims 5 to 9, wherein said detectable grouping is a grouping sensitive to luminescent, colorimetric or radioactive detection.

11. Method according to any of claims 5 to 10, wherein the subunit of EF-1, the homologue of EF-1 as defined in claim 5, or the C-terminal fragment thereof as defined in claim 5, is implemented in a form associated with a GAG viral polyprotein or a homologue thereof, said homologue of GAG having at least 80% homology with GAG and a similar biological activity.

12. Kit for detecting at least one p24 retroviral protein or a homologue thereof, said homologue of p24 having at least 80% homology with p24 and a similar biological activity, comprising:
- at least one first receptacle comprising at least one subunit α of a translation elongation factor 1 protein, EF-1, a homologue of EF-1, said homologue of EF-1 having at least 80% homology with EF-1 and a similar biological activity, or a C-terminal fragment thereof comprising a sequence of amino acids varying from 150 to 250 amino acids contiguous of the C-terminal end of the subunit or its homologue, and
- at least one second receptacle comprising a bifunctional entity provided with a grouping able to bond specifically to at least said p24 retroviral protein or said homologue thereof, liable to be present in a proteic complex formed with the subunit of EF-1, the homologue of EF-1 or the C-terminal fragment thereof, and a grouping suitable for being detectable directly or indirectly by a detection mode.

13. Kit according to the preceding claim, also comprising a GAG viral polyprotein or a homologue thereof, said homologue of GAG having at least 80% homology with GAG and a similar biological activity.
